# EUROPEAN PATENT APPLICATION

(11) **EP 2 845 620 A1**
(43) Date of publication of application: **11.03.2015**
(21) Application number: 14169079.2
(22) Date of filing: 20.05.2014
(51) Int. Cl.: A61M 25/09, D07B 1/12, A61B 17/22, A61M 25/00

(54) **Coil body and medical apparatus using said coil body**

(30) Priority: 06.09.2013 JP 2013184570
(71) Applicant: ASAHI INTECC CO., LTD., Nagoya-shi, Aichi 463-0024 (JP)
(72) Inventor: Matsumoto, Munechika, Nagoya-shi, Aichi 463-0024 (JP); Itou, Katsutoshi, Nagoya-shi, Aichi 463-0024 (JP); Maeda, Takashi, Nagoya-shi, Aichi 463-0024 (JP); Katsuno, Masatoshi, Nagoya-shi, Aichi 463-0024 (JP)
(74) Representative: Winter, Brandl, Fürniss, Hübner, Röss, Kaiser, Polte - Partnerschaft mbB

(57) **Abstract**

[Problem] To provide a coil body and medical apparatus using said coil body which is capable of ensuring the sufficient torque transmissibility.

[Solution] A coil body (30) comprises a first layer (32) which is formed by first wires (32a) wound in a spiral shape, and a second layer (34) which is provided on an outer periphery of the first layer (32) and is formed by second wires (34a) wound in a spiral shape. Furthermore, a tensile strength of first wires (32a) is greater than a tensile strength of second wires (34a).

## Description

### [Technical Field]

The present invention relates to a coil body and medical apparatus using said coil body which are used as a medical member inserted into a body cavity for the purpose of treatment or examination.

### [Background Art]

Conventionally, a variety of configurations have been proposed as a medical apparatus used by being inserted into a body tissue or a tubular organ such as a blood vessel, a digestive tract, or the ureter.

For example, Patent Literature 1 describes a medical apparatus (guide wire) configured of a wire-shaped body molded into a spiral shape to form a reinforcing member which is then arranged on the outer periphery of an inner core. With this reinforcing member, by the side of its proximal end being close-coiled while the side towards the front (side of its distal end) being open-coiled, rigidity is applied to the side of its proximal end while flexibility is applied to the side of its distal end.

### [Citation List]

### [Patent Literature]

[Patent Literature 1] Japanese Unexamined Patent Application Publication No. 1998-99442

When inserting a medical apparatus into a slight gap that exists in a constrictive region of a high degree such as the lesion of a chronic total occlusion, when a rotational torque is applied from the side of the medical apparatus in the hand of the operator, there are cases when it becomes difficult for the medical apparatus to rotate due to the distal end thereof getting caught in the constrictive region. For this reason, in cases when performing treatment for such constrictive region, sufficient torque transmissibility is necessary. However though, with the abovementioned medical apparatus according to Patent Literature 1, due to the open-coiled portion provided on the side of its distal end of the reinforcing member, sufficient torque transmissibility could not be realized which left room for improvement.

### [Summary of Invention]

### [Technical Problem]

The present invention has been devised in light of the abovementioned problem exhibited by conventional technology and the object of the present invention is to provide a coil body and medical apparatus using said coil body which are capable of ensuring sufficient torque transmissibility.

### [Solution to Problem]

In order to solve the abovementioned problem, a coil body according to an embodiment of the present invention has the following characteristics.

Embodiment 1 of the present invention is a coil body comprising a first layer which is formed by a first wire wound in a spiral shape, and a second layer which is provided on an outer periphery of the first layer and is formed by a second wire wound in a spiral shape, wherein a tensile strength of the first wire is greater than a tensile strength of the second wire.

Embodiment 2 of the present invention is the coil body according to Embodiment 1, wherein a third layer formed by a third wire wound in a spiral shape is provided on an outer periphery of the second layer, and the tensile strength of the second wire is greater than a tensile strength of the third wire.

Embodiment 3 of the present invention is the coil body according to Embodiment 1 or Embodiment 2, wherein each of the first wire and the second wire is a twisted wire formed by a plurality of wires twisted together on a substantially same circumference.

Embodiment 4 of the present invention is a medical apparatus characterized by having the coil body according to any of Embodiment 1 through Embodiment 3, a distal end tip formed on a distal end of the coil body, and a gripping portion connected to a side of a proximal end of the coil body.

### [Effects of Invention]

The coil body according to Embodiment 1 comprises a first layer which is formed by a first wire wound in a spiral shape, and a second layer which is provided on an outer periphery of the first layer and is formed by a second wire wound in a spiral shape, while a tensile strength of the first wire is greater than a tensile strength of the second wire. With such a configuration, due to elastic deformation, it is possible for the first wire to act on the second wire on the outer layer from the inner side thereof.

Accordingly, the adhesive force between the first layer and the second layer is increased. As a result, the torque that is generated by rotating the side of the proximal end of the coil body is efficiently transmitted via the second layer and the first layer to the side of the furthest distal end of the coil body. In other words, in such a coil body, it is possible to ensure sufficient torque transmissibility. Due to this, it will be possible to prevent problems where the distal end of the coil body gets caught in a constrictive region.

The coil body according to Embodiment 2 is the coil body according to Embodiment 1 having a third layer formed by a third wire wound in a spiral shape provided on an outer periphery of the second layer, while the tensile strength of the second wire is greater than a tensile strength of the third wire.

With such a configuration, due to elastic deformation, it is possible for the second wires to act on the third wire on the outer layer from the inner side thereof.

As a result, in addition to the adhesive force between the first layer and the second layer, the adhesive force between the second layer and the third layer is increased. Accordingly, the torque that is generated by rotating the side of the proximal end of the coil body is efficiently transmitted via the third layer, the second layer, and the first layer to the side of the furthest distal end of the coil body. In other words, in such a coil body, it is possible to easily ensure further sufficient torque transmissibility.

Each of the first wires and the second wires which form the coil body according to Embodiment 3 is a twisted wire formed by a plurality of wires twisted together on a substantially same circumference. With such a configuration, it is possible to realize relatively minute movement between the wires, and as a result, it is possible to effectively suppress plastic deformation of the coil body and increase the resiliency of the coil body.

The medical apparatus according to Embodiment 4 has the coil body according to any of Embodiment 1 through Embodiment 3, a distal end tip formed on a distal end of the coil body, and a gripping portion connected to a side of a proximal end of the coil body. With such a configuration, it is possible to ensure sufficient torque transmissibility and prevent problems where the distal end of the medical apparatus gets caught in a constrictive region.

### [Brief Description of Drawings]

- [Fig. 1]: Fig. 1 is a schematic view of a coil body according to the first embodiment of the present invention.
- [Fig. 2]: Fig. 2 is a sectional view along A-A of the coil body illustrated in Fig. 1.
- [Fig. 3]: Fig. 3 is a sectional view of a coil body according to the second embodiment of the present invention.
- [Fig. 4]: Fig. 4 is a sectional view of a coil body according to the third embodiment of the present invention.
- [Fig. 5]: Fig. 5 is a schematic view of a coil body according to the fourth embodiment of the present invention.
- [Fig. 6]: Fig. 6 is a sectional view along B-B of the coil body illustrated in Fig. 5.
- [Fig. 7]: Fig. 7 is schematic view of a coil body according to the fifth embodiment of the present invention.
- [Fig. 8]: Fig. 8 is a sectional view along C-C of the coil body illustrated in Fig. 7.
- [Fig. 9]: Fig. 9 is schematic view of a coil body according to the sixth embodiment of the present invention.
- [Fig. 10]: Fig. 10 is a sectional view along D-D of the coil body illustrated in Fig. 9
- [Fig. 11]: Fig. 11 (a) is an overall view of a medical apparatus according to the present invention, while Fig. 11 (b) is a sectional view of the distal end portion of the medical apparatus.

### [Description of Embodiments]

### [First Embodiment]

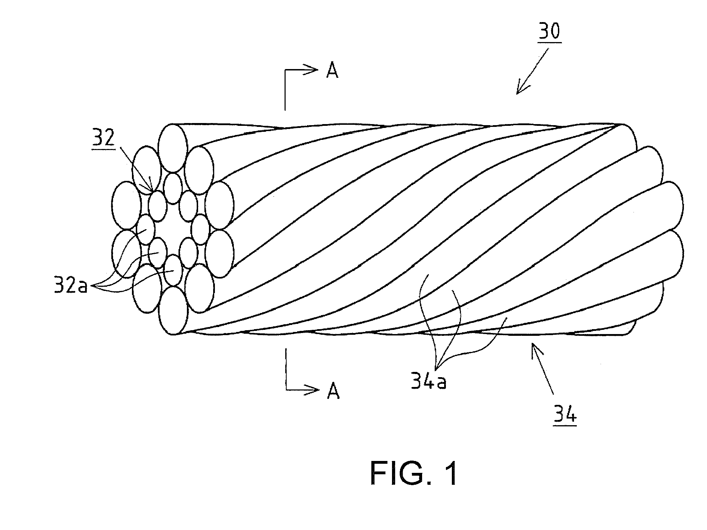

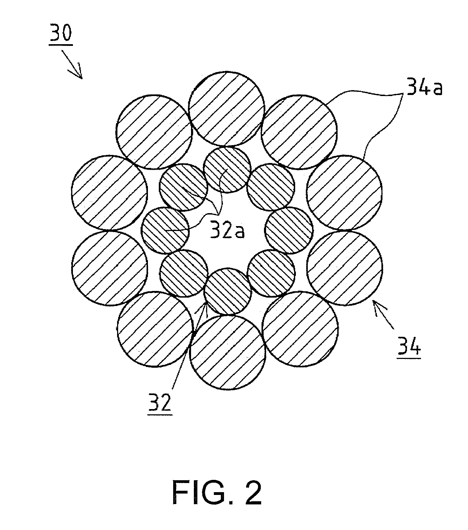

A coil body according to the first embodiment of the present invention will be explained with reference to Fig. 1 and Fig. 2. It shall be noted that each of the drawings schematically illustrates the coil body and thus the dimensional ratios thereof differ from the actual embodiment.

As illustrated in Fig. 1 and Fig. 2, a coil body (30) of this embodiment comprises a first layer (32) formed of a twisted wire formed by a plurality of first wires (32a) twisted together on a substantially same circumference, and a second layer (34) formed of a twisted wire formed by a plurality of second wires (34a) twisted together on a substantially same circumference which is provided on the outer periphery of the first layer (32).

In such a coil body (30), it is possible to realize a relatively minute movement each between first wires (32a) and between second wires (34a), and as a result, since it becomes more difficult for plastic deformation to occur with a coil body (30), it is possible to increase the resiliency of coil body (30).

The tensile strength of the first wires (32a) which form the first layer (32) is greater than the tensile strength of the second wires (34a) which form the second layer (34). In this embodiment, the tensile strength of the first wires (32a) is 2500-2700 N/mm² while the tensile strength of the second wires (34a) is 2100-2400 N/mm². Furthermore, the first wires (32a) which form the first layer (32) and the second wires (34a) which form the second layer (34) are in close contact with each other.

By the tensile strength of the first wires (32a) which form the first layer (32) being greater than the tensile strength of the second wires (34a) which form the second layer (34), due to elastic deformation, it becomes possible for the first wires (32a) to act on the second wires (34a) on the outer layer from the inner side thereof. Therefore, the adhesive force between the first layer (32) and the second layer (34) is increased.

As a result, the torque that is generated by rotating the side of the proximal end of the coil body (30) is efficiently transmitted via the second layer (34) and the first layer (32) to the side of the furthest distal end of the coil body (30). In other words, in such a coil body (30), it is possible to ensure the sufficient torque transmissibility. Due to this, it becomes possible to prevent problems where the distal end of the coil body (30) gets caught in a constrictive region.

As materials used to form the first wires (32a) and the second wires (34a), although for example stainless steel, tungsten, or Ni-Ti alloys, etc. can be listed, the materials used are not specifically limited to these as long as the tensile strength of the first wires (32a) is greater than the tensile strength of the second wires (34a). In this embodiment, for example SUS304WPS is used as the material to form the first wires (32a) while for example SUS304WPB is used as the material to form the second wires (34a).

It is preferable that the diameter of the first wires (32a) is smaller than the diameter of the second wires (34a). In this embodiment, the diameter of the first wires (32a) is approximately 0.06 mm while the diameter of the second wires (34a) is approximately 0.07 mm. With such a configuration, the gap between the second wires (34a) and the first wires (32a) can be made smaller, thus increasing the adhesive force between the first layer (32) and the second layer (34). As a result, it is possible to further improve the torque transmissibility of coil body (30).

In addition, the winding direction of the first wires (32a) and the winding direction of the second wires (34a) are in opposite directions. With such a configuration, for example, the torque transmissibility when the coil body (30) is rotated in the clockwise direction and the torque transmissibility when the coil body (30) is rotated in the counter-clockwise direction become roughly equivalent. In other words, it is possible to easily ensure the torque transmissibility that is well balanced along the circumferential cross section. Accordingly, it is possible to increase the operability when the coil body (30) is inserted deep inside a blood vessel.

Here, in this embodiment, although the diameter of the first wires (32a) is configured to be smaller than the diameter of the second wires (34a), the relationship between the diameters of each of these wires is not limited to the above configuration. In other words, the diameter of the first wires (32a) may be substantially identical to the diameter of the second wires (34a), or the diameter of the first wires (32a) may be larger than the diameter of the second wires (34a).

### [Second Embodiment]

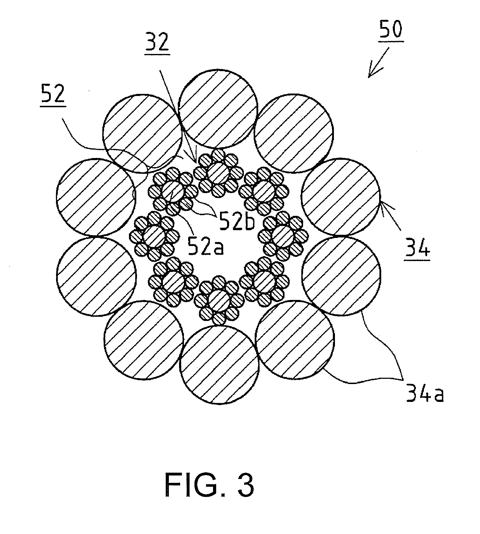

A coil body according to the second embodiment of the present invention will be explained with reference to Fig. 3. It shall be noted that configuring components identical to those in the abovementioned embodiment will be assigned an identical reference sign while the explanation of such will be omitted as the following explanation will focus on the points that are different.

In the abovementioned first embodiment, a first layer (32) was formed of a twisted wire formed by a plurality of first wires (32a) twisted together on a substantially same circumference. Contrary to this, the first layer (32) which forms a coil body (50) according to the second embodiment is formed by a first wire member (52) consisting of a first core wire (52a) and 8 strands of first side wires (52b), wound such that the outer periphery of the first core wire (52a) is covered, twisted together on a substantially same circumference.

Here, as materials used to form the first core wire (52a) and the first side wires (52b), although for example stainless steel, tungsten, or Ni-Ti alloys, etc. can be listed, the materials used are not specifically limited to these as long as the tensile strength of first wire member (52) on the inner side is greater than the tensile strength of second wires (34a).

In the second embodiment, apart from being able to realize effects similar to the abovementioned first embodiment, it is possible to realize a relatively minute movement between the first side wires (52b) in the first layer (32), and as a result, it is possible to effectively suppress plastic deformation of coil body (50) and further increase the resiliency of coil body (50).

In addition, in this embodiment, although the coil body (50) is formed with the 2 layers of the first layer (32) and the second layer (34) on the outer periphery thereof, the number of layers is not limited to the above configuration. In other words, the coil body (50) may be formed of 3 or more layers. Even in such case, in order to increase the torque transmissibility of coil body (50), it is preferable that the tensile strength of each layer is greater in order of those positioned as the inner layers.

Furthermore, as long as it is possible to maintain the shape of coil body (50), the first core wire (52a) of the first layer (32) may be omitted. In other words, it is possible to use a configuration in which the first wire member (52) is hollow-shaped.

### [Third Embodiment]

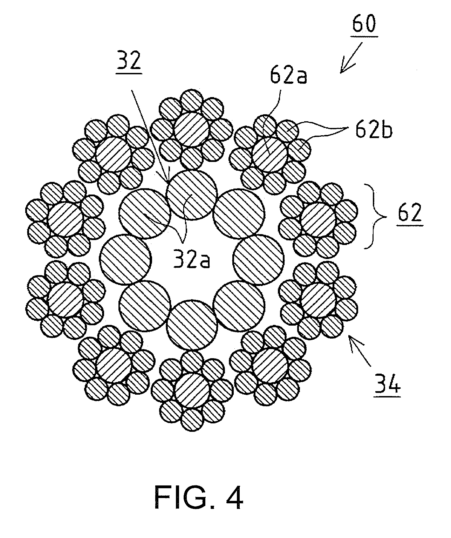

A coil body according to the third embodiment of the present invention will be explained with reference to Fig. 4. It shall be noted that configuring components identical to those in the abovementioned embodiments will be assigned an identical reference sign while the explanation of such will be omitted as the following explanation will focus on the points that are different.

In the abovementioned first embodiment, the second layer (34) was formed of a twisted wire formed by a plurality of second wires (34a) twisted together on a substantially same circumference. Contrary to this, the second layer (34) which forms the coil body (60) according to the third embodiment is formed by second wire member (62) consisting of a second core wire (62a) and 8 strands of second side wires (62b), wound such that the outer periphery of second core wire (62a) is covered, twisted together on a substantially same circumference.

As materials used to form the second core wire (62) and the second side wires (62b), although for example stainless steel, tungsten, or Ni-Ti alloys, etc. can be listed, the materials used are not specifically limited to these as long as the tensile strength of first wires (32a) on the inner side is greater than the tensile strength of second wire member (62).

In the third embodiment, apart from being able to realize effects similar to the abovementioned first embodiment, it is possible to realize a relatively minute movement between the second side wires (62b) in the second layer (34), and as a result, it is possible to effectively suppress plastic deformation of coil body (60) and further increase the resiliency of coil body (60).

In addition, in this embodiment, although the coil body (60) is formed with the 2 layers of the first layer (32) and the second layer (34) on the outer periphery thereof, the number of layers is not limited to the above configuration. In other words, the coil body (60) may be formed of 3 or more layers. Even in such case, in order to increase the torque transmissibility of coil body (60), it is preferable that the tensile strength of each layer is greater in order of those positioned as the inner layers.

Furthermore, as long as it is possible to maintain the shape of coil body (60), the second core wire (62a) of the second layer (34) may be omitted. In other words, it is possible to use a configuration in which the second wire member (62) is hollow-shaped.

### [Fourth Embodiment]

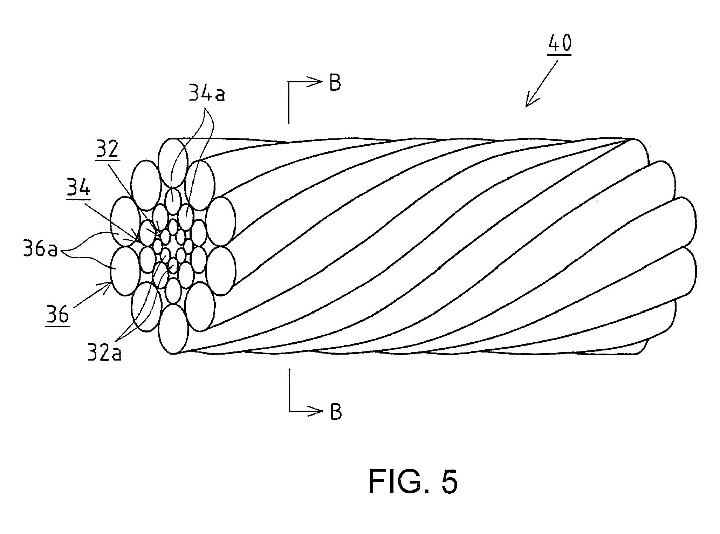

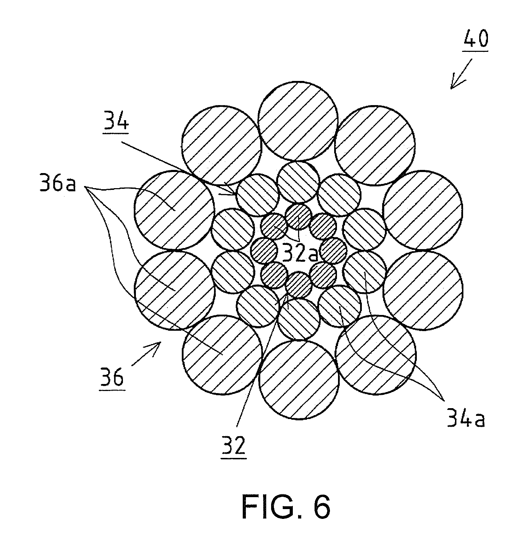

A coil body according to the fourth embodiment of the present invention will be explained with reference to Fig. 5 and Fig. 6. It shall be noted that configuring components identical to those in the abovementioned embodiments will be assigned an identical reference sign while the explanation of such will be omitted as the following explanation will focus on the points that are different.

In a coil body (40) according to the fourth embodiment, the third layer (36) formed of a twisted wire formed by a plurality of third wires (36a) twisted together on a substantially same circumference is provided on the outer periphery of second layer (34) in the first embodiment. The tensile strength of the second wires (34a) which form second layer (34) is greater than the tensile strength of third wires (36a) which form the third layer (36). It shall be noted that in this embodiment, the tensile strength of third wires (36a) is 1600-2000 N/mm².

With such a configuration, due to elastic deformation, it becomes possible for the second wires (34a) to act on the third wires (36a) on the outer layer from the inner side thereof. As a result, in addition to the adhesive force between the first layer (32) and the second layer (34), the adhesive force between the second layer (34) and the third layer (36) is increased.

Furthermore, the torque that is generated by rotating the side of the proximal end of coil body (40) is efficiently transmitted via the third layer (36), the second layer (34), and the first layer (32) to the side of the furthest distal end of coil body (40). In other words, in such a coil body (40), it is possible to easily ensure the further sufficient torque transmissibility.

As the material used to form the third wires (36a), although for example stainless steel, tungsten, or Ni-Ti alloys, etc. can be listed, the material used is not specifically limited to these as long as the tensile strength of second wires (34a) is greater than the tensile strength of third wires (36a). In this embodiment, the stainless steel (SUS304-3/4H) is used as the material to form the third wires (36a).

In addition, in this embodiment, although the coil body (40) is formed with the 3 layers of the first layer (32), the second layer (34) provided on the outer periphery of first layer (32), and the third layer (36) provided on the outer periphery of second layer (34), the number of layers is not limited to the above configuration. In other words, the coil body (40) may be formed of 4 or more layers. Even in such case, in order to increase the torque transmissibility of coil body (40), it is preferable that the tensile strength of each layer is greater in order of those positioned as the inner layers.

### [Fifth Embodiment]

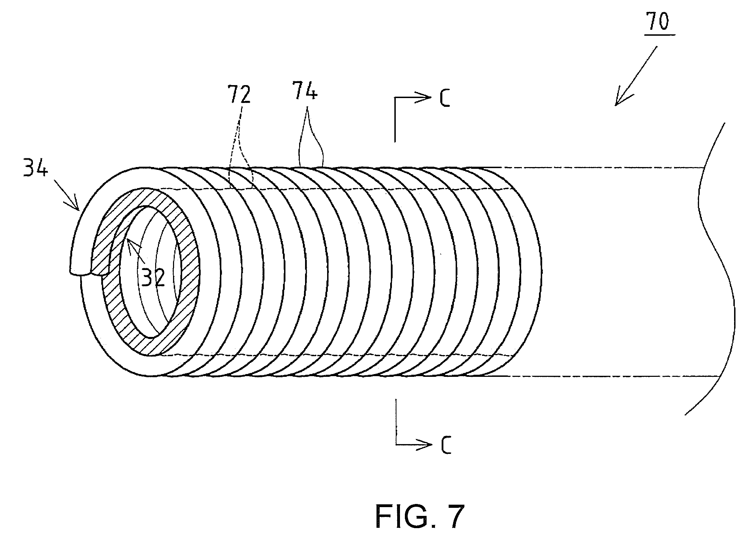

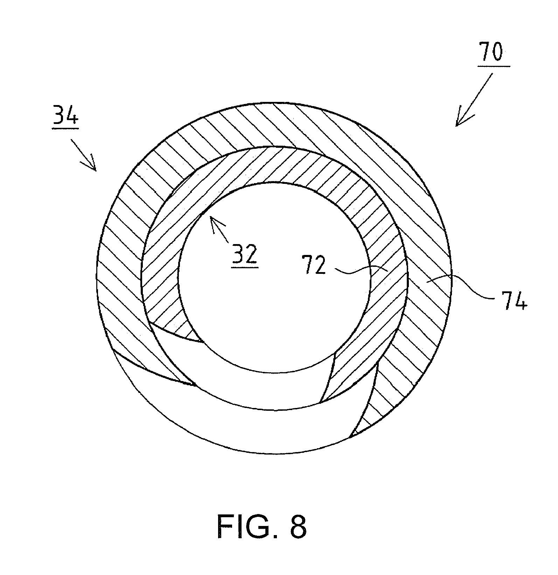

A coil body according to the fifth embodiment of the present invention will be explained with reference to Fig. 7 and Fig. 8. It shall be noted that configuring components identical to those in the abovementioned embodiments will be assigned an identical reference sign while the explanation of such will be omitted as the following explanation will focus on the points that are different.

In the abovementioned first embodiment, the first layer (32) was formed of a twisted wire formed by a plurality of first wires (32a) twisted together on a substantially same circumference while the second layer (34) was formed of a twisted wire formed by a plurality of second wires (34a) twisted together on a substantially same circumference. Contrary to this, a coil body (70) according to the fifth embodiment comprises the first layer (32) formed of the first wire (72) wound in a spiral shape, and the second layer (34) formed of the second wire (74) wound in a spiral shape. In other words, in this embodiment, the first layer (32) and the second layer (34) are each configured of a single wire coil formed by a single wire wound in a spiral shape.

Furthermore, the tensile strength of first wire (72) is greater than the tensile strength of second wire (74). With such a configuration, due to elastic deformation, it becomes possible for the first wire (72) to act on the second wire (74) on the outer layer from the inner side thereof. Accordingly, the adhesive force between the first layer (32) and the second layer (34) is increased.

As a result, the torque that is generated by rotating the side of the proximal end of coil body (70) is efficiently transmitted via the second layer (34) and the first layer (32) to the side of the furthest distal end of coil body (70). In other words, in such a coil body (70), it is possible to ensure the sufficient torque transmissibility. Due to this, it becomes possible to prevent problems where the side of the distal end of coil body (70) gets caught in a constrictive region.

Furthermore, as materials used to form the first wire (72) and the second wire (74), although for example stainless steel, tungsten, or Ni-Ti alloys, etc. can be listed, the materials used are not specifically limited to these as long as the tensile strength of first wire (72) on the inner side is greater than the tensile strength of second wire (74) on the side of the outer layer.

### [Sixth Embodiment]

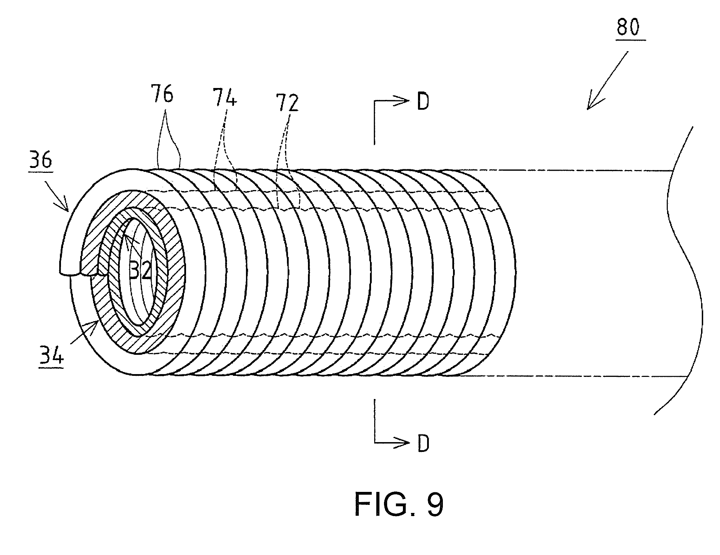

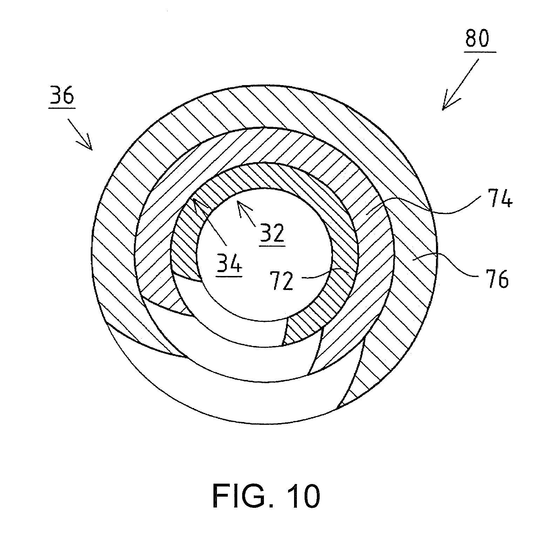

A coil body according to the sixth embodiment of the present invention will be explained with reference to Fig. 9 and Fig. 10. It shall be noted that configuring components identical to those in the abovementioned embodiments will be assigned an identical reference sign while the explanation of such will be omitted as the following explanation will focus on the points that are different.

A coil body (80) according to the sixth embodiment is configured having the third layer (36), formed of a third wire (76) wound in a spiral shape, provided on the outer periphery of the second layer (34) of the abovementioned coil body (70) according to the fifth embodiment. In other words, in this embodiment, the first layer (32), the second layer (34), and the third layer (36) are each configured of a single wire coil formed by a single wire wound in a spiral shape.

Furthermore, in this embodiment, the tensile strength of second wire (74) is greater than the tensile strength of third wire (76). With such a configuration, due to elastic deformation, it becomes possible for the second wire (74) to act on the third wire (76) on the outer layer from the inner side thereof.

As a result, in addition to the adhesive force between the first layer (32) and the second layer (34), the adhesive force between the second layer (34) and the third layer (36) is increased. Accordingly, the torque that is generated by rotating the side of the proximal end of coil body (80) is efficiently transmitted via the third layer (36), the second layer (34), and the first layer (32) to the side of the furthest distal end of coil body (80). In other words, in such a coil body (80), it is possible to easily ensure the further sufficient torque transmissibility.

Furthermore, as the material used to form the third wire (76), although for example stainless steel, tungsten, or Ni-Ti alloys, etc. can be listed, the material used is not specifically limited to these as long as the tensile strength of second wire (74) on the side of the inner layer is greater than the tensile strength of third wire (76).

In addition, in this embodiment, although the coil body (80) is formed with the 3 layers of the first layer (32), the second layer (34) provided on the outer periphery of first layer (32), and the third layer (36) provided on the outer periphery of the second layer (34), the number of layers is not limited to the above configuration. In other words, the coil body (80) may be formed of 4 or more layers. Even in such case, in order to increase the torque transmissibility of coil body (80), it is preferable that the tensile strength of each layer is greater in order of those positioned as the inner layers.

### [Seventh Embodiment]

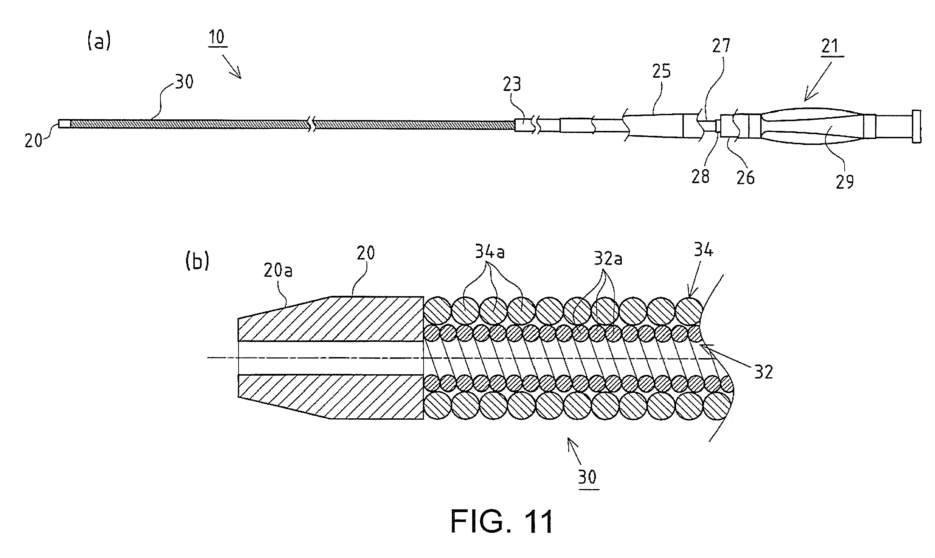

Fig. 11 (a) is an overall view of a medical apparatus for atherectomy which uses a coil body according to the present invention. In Fig. 11(a), the left side of the drawing is the side of the distal end which is inserted into the body cavity while the right side of the drawing is the side of the proximal end which is operated by an operator such as a doctor. In addition, Fig. 11 (b) is a sectional view of the distal end portion of this medical apparatus. Furthermore, this Fig. 11 (b) illustrates a medical apparatus that uses a coil body (30) of the first embodiment.

As illustrated in Fig. 11 (a) and Fig. 11 (b), medical apparatus (10) comprises a coil body (30), a distal end tip (20) which is a tubular and joined to the distal end of coil body (30), and a gripping portion (21) provided on the side of the proximal end of coil body (30).

As illustrated in Fig. 11 (a), a distal end tip (20), for example formed of platinum, has a tapered portion (20a) with a diameter that decreases toward the tip. Furthermore, a screw-like uneven texture may be provided on the inner peripheral surface or the outer peripheral surface of the distal end tip (3).

The coil body (30) has the proximal end portion (rear end portion) thereof covered with a cover tube (23). A round protection pipe (27), for example made of stainless steel, is inserted and fitted in the proximal end portion of the cover tube (23). A square protection pipe (28) is externally fitted on the proximal end portion of this round protection pipe (27).

Meanwhile, a gripping portion (21) is formed of protector (25) which is cylindrical with the side of the distal end thereof forming a tapered shape having a narrowed tip and cylindrical connector (29) having smaller radius distal end portion (26) fitted on the rear end portion of protector (25) and fins (F, F) on the outer periphery. A smaller radius distal end portion (26) is externally fitted on square protection pipe (28) while a gripping portion (21) is configured to rotate as a single unit with the rear end portion of coil body (30).

Medical apparatus (10) is, for example in an operation to bore the lesion of such as the closed portion of a coronary artery, operated by pressing and twisting the gripping portion (21) from a state where the apparatus is inserted into the blood vessel and the distal end tip (3) is poking against the lesion. During this operation, a rotation is applied to the coil body (30) and a screwing effect is exhibited when the distal end tip (3) comes into contact with the lesion. Furthermore, depending on the direction in which the gripping portion (21) is rotated, it is possible to screw in or remove the medical apparatus (10).

When the above operation is performed, the torque that is generated by rotating the gripping portion (21) of medical apparatus (10) is efficiently transmitted via the second layer (34) and the first layer (32) of the coil body (30) to the furthest distal end portion of medical apparatus (10). In other words, in such medical apparatus (10) using the coil body (30), it is possible to ensure the sufficient torque transmissibility. Due to this, it becomes possible to prevent problems where the distal end of medical apparatus (10) gets caught in a constrictive region.

Furthermore, in this embodiment, although a medical apparatus using the coil.body (30) of the first embodiment was explained, even with medical apparatus using any one of coil body (40, 50, 60, 70, 80) according to the second through sixth embodiments, it is possible to realize effects similar to that of this embodiment.

### [Reference Signs List]

- 10: Medical apparatus
- 20: Distal end tip
- 21: Gripping portion
- 30, 40, 50, 60, 70, 80: Coil body
- 32a, 72: First wire
- 32: First layer
- 34a, 74: Second wire
- 34: Second layer
- 36a, 76: Third wire
- 36: Third layer

## Claims

1. A coil body (30, 40, 50, 60, 70, 80) comprising:
a first layer (32) which is formed by a first wire (32a, 52, 72) wound in a spiral shape, and
a second layer (34) which is provided on an outer periphery of the first layer (32) and is formed by a second wire (34a, 62, 74) wound in a spiral shape, wherein
a tensile strength of the first wire (32a, 52, 72) is greater than a tensile strength of the second wire (34a, 62, 74).

2. A coil body (40, 80) according to Claim 1, wherein
a third layer (36) formed by a third wire (36a, 76) wound in a spiral shape is provided on an outer periphery of the second layer (34a, 62, 74), and
the tensile strength of the second wire (34a, 62, 74) is greater than a tensile strength of the third wire (36a, 76).

3. A coil body (30, 40, 50, 60) according to Claim 1 or Claim 2, wherein
each of the first wire (32a, 52) and the second wire (34a, 62) is a twisted wire formed by a plurality of wires twisted together on a substantially same circumference.

4. A medical apparatus (10) **characterized by** having the coil body (30, 40, 50, 60, 70, 80) according to any of Claims 1 through 3, a distal end tip (20) formed on a distal end of the coil body, and a gripping portion (21) connected to a side of a proximal end of the coil body.
